# EUROPEAN PATENT SPECIFICATION

(11) **EP 4 376 947 B1**
(45) Date of publication and mention of the grant of the patent: **16.09.2026**
(21) Application number: 22754420.2
(22) Date of filing: 26.07.2022
(51) Int. Cl.: A61N 5/10

(54) **AN APPARATUS AND A COMPUTER IMPLEMENTED METHOD FOR CALCULATING A RADIOTHERAPY DOSE DISTRIBUTION USING MONTE CARLO SIMULATIONS**
VORRICHTUNG UND COMPUTERIMPLEMENTIERTES VERFAHREN ZUR BERECHNUNG EINER RADIOTHERAPEUTISCHEN DOSISVERTEILUNG UNTER VERWENDUNG VON MONTE-CARLO-SIMULATIONEN
APPAREIL ET PROCÉDÉ MIS EN UVRE PAR ORDINATEUR POUR CALCULER UNE DISTRIBUTION DE DOSE DE RADIOTHÉRAPIE UTILISANT DE SIMULATIONS DE MONTE-CARLO

(30) Priority: 27.07.2021 EP 21187829
(43) Date of publication of application: 05.06.2024
(73) Proprietor: Smart Scientific Solutions B.V., 6229 EV Maastricht (NL)
(72) Inventor: STAUT, Nick Josef, 6629 EV MAASTRICHT (NL); VAN DIJK, Robert Harald Wiljan, 6629 EV MAASTRICHT (NL); VERHAEGEN, Frans Jozefina Willem, 6629 EV MAASTRICHT (NL)
(74) Representative: Algemeen Octrooi- en Merkenbureau B.V.
(86) International application number: PCT/EP2022/070900
(87) International publication number: WO 2023/006720

(56) References cited:
- EP-A1- 3 695 882
- US-A1- 2018 085 599
- US-A1- 2020 065 940
- RYAN NEPH ET AL: "DeepMC: a deep learning method for efficient Monte Carlo beamlet dose calculation by predictive denoising in magnetic resonance-guided radiotherapy", PHYSICS IN MEDICINE AND BIOLOGY, INSTITUTE OF PHYSICS PUBLISHING, BRISTOL GB, vol. 66, no. 3, 28 January 2021 (2021-01-28), pages 35022, XP020359779, ISSN: 0031-9155, [retrieved on 20210128], DOI: 10.1088/1361-6560/ABCA01
- GAO MINGJIE ET AL: "Deep Convolutional Neural Network With Adversarial Training for Denoising Digital Breast Tomosynthesis Images", IEEE TRANSACTIONS ON MEDICAL IMAGING, IEEE, USA, vol. 40, no. 7, 17 March 2021 (2021-03-17), pages 1805 - 1816, XP011863557, ISSN: 0278-0062, [retrieved on 20210630], DOI: 10.1109/TMI.2021.3066896

## Description

### TECHNICAL FIELD

The present disclosure relates to the field of radiation dose distribution planning techniques, for example in clinical radiotherapy treatment planning systems. In particular, the present disclosure pertains to an apparatus and a computer implemented method for calculating a radiotherapy dose distribution using Monte Carlo simulations passing through a target region or region of interest within a target object.

### BACKGROUND OF THE INVENTION

In the past decades, the sophistication of dose calculation models implemented in clinical radiotherapy treatment planning systems has gradually improved, together with available computing power in hospitals. This evolution, going from rather simple scatter and inhomogeneity corrections to pencil beams and superposition / convolution models has resulted in continuous improvements in the accuracy of predicted patient doses. In superposition / convolution models, predetermined Monte Carlo results are used. Full Monte Carlo dose calculations would therefore seem the next logical step.

In a Monte Carlo dose calculation, the track of each individual ionizing particle (in radiotherapy generally photons, electrons and protons) through the volume of interest is simulated. Along its way, the particle may interact with the matter through which it is passing, e.g. through Compton scattering (for photons) or Coulomb scattering (for electrons and protons). For a dose calculation, for each interaction that is simulated, the energy balance is calculated of the energy of the 'incoming' particle(s) minus the energy of the 'outgoing' one(s). To calculate the dose in a particular volume (voxel), the contributions from all interactions taking place inside the volume are added, and divided by the mass in the volume.

For many years it has been realised that full Monte Carlo simulations of the radiotherapy dose delivery process should further improve calculation accuracy. Due to limitations in computing power, however, this was never a realistic option in a clinical setting. Today, available computing power may still not allow for full Monte Carlo simulations in clinical practice. Approximations and simplifications to speed up the calculations may therefore be necessary, possibly (partially) jeopardising the advantages of full Monte Carlo dose calculations.

Examples of such methods are provided in US 2020/065940 A1 and EP 3695882 A1 as captured with the preamble of claim 1 of this disclosure.

However, given the random nature of Monte Carlo dose calculations, the resulting dose distributions are composed of noisy dose data and associated statistical uncertainty data (noise), which prevents making reliable clinical decisions within short time spans desirable for using or implementing such resulting dose distributions as a setting in a radiation treatment system for the purpose of radiation therapy in a target region or region of interest in, for example, a human or animal body or an inanimate body.

### SUMMARY OF THE INVENTION

The invention is set out in the appended set of claims. According to a first aspect of the disclosure as completely captured in claim 1 pertains to a computer implemented method for calculating and displaying, within a computer-generated display of a display device, a radiation dose distribution of a radiation beam using Monte Carlo simulations passing through a target region or region of interest within a target object, the computer implemented method comprising at least the steps of:
i) obtaining a set of three-dimensional image data representing the region of interest in the target object;
ii) generating a display signal causing the display device to display the three-dimensional image data set of the region of interest on the display device,
iii) receiving a user selection, based on user interaction with the three-dimensional image data set on the display device, the user selection identifying one or more selected locations of interest in the displayed three-dimensional image set;
iv) calculating, using radiation dose calculation means, a radiation dose distribution using Monte Carlo simulation of a radiation beam passing through the region of interest taking the one or more selected locations of interest into account, the radiation dose distribution being composed of noisy dose data and associated uncertainty data;
v) denoising the radiation dose data distribution for the associated uncertainty data with one or more trained machine learning algorithms, thereby generating a denoised radiation dose distribution.

As outlined, the calculated radiation dose data distribution using Monte Carlo simulations contains next to a noisy dose data set also an associated uncertainty data set, clinical decision making at a short time interval is highly preferably in view of performing real-time radiation therapy treatments on a human or animal body.

Accordingly, by denoising the calculated radiation dose data distribution as obtained using Monte Carlo simulations for the associated uncertainty data set using one or more trained machine learning algorithms, a denoised radiation dose distribution can be generated with a significant gain in calculation time, and accordingly the computer implemented method can be used for performing real-time radiation therapy treatments.

In a preferred example of the computer-implemented method according to the disclosure, the computer implemented method further comprises the step of:
vi) displaying, within the three-dimensional image data set of the region of interest displayed the display device, the generated denoised radiation dose data distribution.

Furthermore, in preferred examples, the one or more machine learning algorithms are selected from the group exemplified by but not limited to an artificial neural network, a decision tree, a regression model, a k-nearest neighbour model, a partial least squares model, a support vector machine, or an ensemble of the models that are integrated to define an algorithm.

Preferably, the one or more machine learning algorithms is a computer-implemented artificial neural network, and whereas, for training the computer-implemented artificial neural network, the computer implemented method further comprises the steps of:
A) inputting, to the computer-implemented artificial neural network, training region of interest data, training noisy dose data, training associated uncertainty data and training denoised dose data characterizing at least one training radiation dose distribution of a radiation beam passing through the training region of interest and one or more selected training locations of interest in the training region of interest;
B) applying, to the computer-implemented artificial neural network, test region of interest data, test noisy dose data and test associated uncertainty data characterizing a test radiation dose distribution of a radiation beam passing through a test region of interest and one or more selected test locations of interest in the test region of interest;
C) analyzing each applied test radiation dose distribution using the at least one training radiation dose distribution to generate a denoised radiation dose distribution for each test noisy dose data and test associated uncertainty data.

Accordingly, the artificial neural network is trained on the basis of machine learning techniques, in particular deep learning, for example by back propagation. The inputted training data used can be classified in three types of data sets, that is noisy dose data set, and the associated statistical uncertainty data (noise) set, both data sets obtained using Monte Carlo simulations over a limited or short calculation time period, as well as an associated denoised dose data set. Preferably, the associated denoised dose data set represents a radiation dose distribution of a radiation beam calculated using Monte Carlo simulations over an extended or longer calculation time period wherein the associated statistical uncertainty data (noise) set is less significant.

In an example, the computer-implemented artificial neural network is a deep learning neural network comprising 2D, and/or 3D convolutional layers, and/or recurrent layers.

According to the disclosure, also an apparatus is proposed for calculating and displaying a radiation dose distribution of a radiation beam using Monte Carlo simulations passing through a region of interest within a target object , the apparatus comprises:
- image data processing means for obtaining a set of image data representing the region of interest;
- selecting means for selecting one or more selected locations of interest within the three-dimensional image set;
- radiation dose calculation means for calculating a radiation dose data distribution using Monte Carlo simulation of a simulated radiation beam passing through the region of interest, wherein the calculated radiation dose data distribution being composed of noisy dose data and associated uncertainty data and wherein
the radiation dose calculation means are further configured in denoising the radiation dose data distribution for the associated uncertainty data using one or more trained machine learning algorithms, thereby generating a denoised radiation dose distribution.

Accordingly, as with the computer implemented method according to the disclosure, as the apparatus is arranged in denoising the calculated radiation dose data distribution as obtained using Monte Carlo simulations for the associated uncertainty data set using one or more trained machine learning algorithms, a denoised radiation dose distribution can be generated with a significant gain in calculation time, and accordingly the computer implemented method can be used for performing real-time radiation therapy treatments.

Herewith, expensive and specialized radiation therapy treatment equipment requiring intensive computing power is avoided. The apparatus according to an example of the disclosure can be simplified as it requires less complex equipment, and it allows for a significant speed up of the dose distribution calculations, whilst maintaining dose distribution accuracy and thus still making reliable clinical decisions.

In a further example, the apparatus also comprises a display device for displaying the three-dimensional image data set of the region of interest and the denoised radiation dose data distribution in the three-dimensional image data set.

Preferably, the one or more machine learning algorithms are selected from the group exemplified by but not limited to an artificial neural network, a decision tree, a regression model, a k-nearest neighbour model, a partial least squares model, a support vector machine, or an ensemble of the models that are integrated to define an algorithm.

In an advantageous example of the apparatus according to the disclosure, the one or more machine learning algorithms is a computer-implemented artificial neural network, and wherein the radiation dose calculation means comprises a training unit to train the computer-implemented artificial neural network, the training unit being configured to:
A) input, to the computer-implemented artificial neural network, training region of interest data, training noisy dose data, training associated uncertainty data and training denoised dose data characterizing at least one training radiation dose distribution of a radiation beam passing through the training region of interest and one or more selected training locations of interest in the training region of interest;
B) apply, to the computer-implemented artificial neural network, test region of interest data, test noisy dose data and test associated uncertainty data characterizing a test radiation dose distribution of a radiation beam passing through a test region of interest and one or more selected test target locations in the test region of interest;
C) analyze each applied test radiation dose distribution using the at least one training radiation dose distribution to generate a denoised radiation dose distribution for each test noisy dose data and test associated uncertainty data.

Using a trained artificial neural network allows for a proper and accurate calculation of a denoised radiation dose distribution for radiation therapy treatment purposes, which calculation is significantly speed up in time. This allows for a reliable clinical decision at short time intervals, which is highly preferably in view of performing real-time radiation therapy treatments on a human or animal body.

In particular, herewith a denoised radiation dose distribution can be calculated over a significant limited or significant shorter calculation time period, compared to the usual radiation dose distribution calculation, which requires Monte Carlo simulations over an extended or longer calculation time period.

In an example of the apparatus, the computer-implemented artificial neural network is a deep neural network comprising 2D, and/or 3D convolutional layers, and/or recurrent layers.

For example, the computer implemented method of the present disclosure can be embodied in a computer program or product, which computer program or product comprises computer-coded instructions which, when the computer program or product program is executed by a computer, such as a laptop or a computer, cause the computer to carry out steps of the computer implemented method disclosed herein.

In a particular embodiment, a computer-readable storage medium is proposed comprising computer-coded instructions stored therein, which computer-coded instructions, when executed by a computer, causes the computer to carry out steps of the computer implemented method disclosed in this application. Such computer-readable storage medium can be a (solid-state) hard drive, a USB drive, or a (digital) optical disc.

### BRIEF DESCRIPTION OF THE DRAWINGS

The invention will now be discussed with reference to the drawings, which show in:
Figure 1 a non-limiting example of the steps for calculating a radiation dose distribution of a radiation beam passing through a target region or region of interest within a target object, for example for radiotherapy treatment;
Figure 2 a non-limiting example of the steps for calculating a radiation dose distribution of a radiation beam using Monte Carlo simulations according to the state of the art;
Figure 3 a non-limiting example of the steps for calculating a radiation dose distribution of a radiation beam using Monte Carlo simulations with the use of the computer implemented method according to the invention;
Figures 4A and 4B further details of an example of both the computer implemented method and an apparatus according to the invention;
Figure 5 another detailed example of an apparatus according to invention, implementing an example of the computer implemented method according to the invention, based on a computer-implemented artificial neural network.

### DETAILED DESCRIPTION OF THE INVENTION

For a proper understanding of the invention, in the detailed description below corresponding elements or parts of the invention will be denoted with identical reference numerals in the drawings.

It is known in the prior art that for dose calculation models implemented in clinical radiotherapy treatment planning systems suitable dose planning software is used. The use of such radiation treatment planning software system allows medical personal to prepare and conduct the intended radiotherapy treatment on a simulated target region or region of interest in and of a target object. The target object can be, for example, a phantom body, or a human or animal body.

Usually, the simulated region of interest of the target object is stored in the form of digital region of interest data into the radiation treatment planning software system, which executes the doses planning software. Preferably, the digital region of interest data representing the simulated region of interest of the target object is acquired by means of suitable imaging techniques, such as CT- and/or MRI-scanning systems. In particular CT- and/or MRI-scanning systems can be used, when the simulated region of interest is part of a real target object such as a human or animal body for the purpose of an actual radiotherapy treatment.

Usually, multiple 2D image slices of the target body are acquired, which are combined in a 3D image data set or image representation, which is stored in into the radiation treatment planning software system.

The digital region of interest data representing the stored 3D image data simulation of the region of interest of the target object include parameters or data relating to the outer contours and the structure (density, different materials or tissues) of the target body, the exact locations of certain locations of interest within the region of interest. The locations of interest can encompass internal organs, such as lungs, heart, intestines, urinary bladder, reproductive organs, etc. as well as the bone structure. Another, often important location of interest within the region of interest can be tumour tissue, in particular its location and its size inside the target body.

This region of interest data together with target body specific parameters, such as the gender, age and dimensions of the patient in the event the target body pertains to a human or animal body are used by the dose planning software program loaded in the radiation treatment planning software system.

In addition, specific characteristic data as to the radiation emitting device used are entered into said dose planning software program and used for the proper calculation of the radiation dose distributions and subsequent planning of a radiation therapy treatment solution for the intended radiation therapy treatment of the tumour.

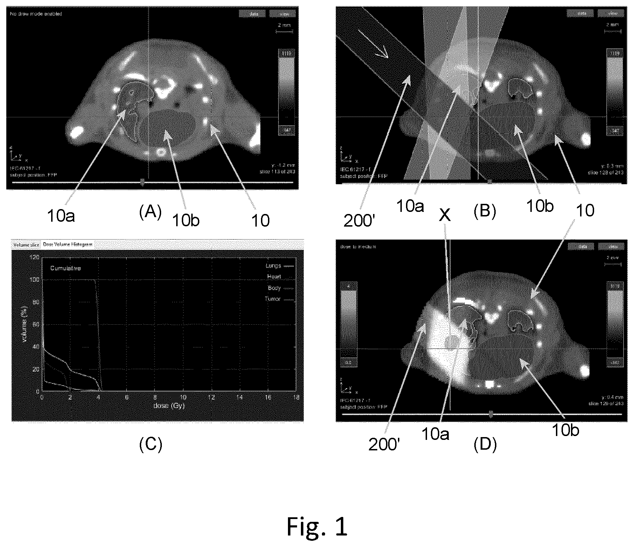

An example of these preparatory steps for calculating a radiation dose distribution and subsequent radiation therapy treatment solution in a known radiation treatment planning software system or apparatus is depicted in Figures 1A-1D.

In Figure 1A, a 2D image slice is part of stored 3D image data, which is a complete simulation or representation of the region of interest of the target object 10, here the body of an animal, in particular a rodent. The stored 3D image data set is displayed by suitable imaging means on a display screen or device. The region of interest data as obtained with suitable imaging techniques, such as CT- and/or MRI-scanning systems, represent and visualize this particular 2D image slice of the region of interest of the target body 10. It depicts also specific locations of interest 10a and 10b, representing e.g. a lung, the heart, intestines, and bones (ribs).

Next, see Figure 1B, one or more locations of interest 10a and 10b are selected in the displayed three-dimensional image set of the region of interest of the target body 10. Such locations of interest 10a and 10b are selected by a user, such as skilled medical personnel operating the radiation treatment planning software system. Based on this user interaction with the three-dimensional image data set on the display device, the user selection identifies these multiple locations of interest 10a and 10b, and also can input information as to an intended or projected radiation beam 200' passing the region of interest 10.

Together with additional region of interest data (density, different materials or tissues, etc.) as well as with target body specific parameters mentioned above, the dose planning software program or radiation dose calculation means calculate a radiation dose distribution, see Figures 1C and 1D. In Figure 1D, reference numeral X denotes the location (e.g. the simulated tumor) within the region of interest 10 receiving the maximum radiation dose.

In the past decades, the sophistication of dose calculation models implemented in clinical radiotherapy treatment planning systems has gradually improved, together with available computing power in hospitals. In superposition/convolution models, predetermined Monte Carlo results are used. Full Monte Carlo dose calculations would therefore seem the next logical step.

In a Monte Carlo dose calculation, the track of each individual ionizing particle (in radiotherapy generally photons, electrons and protons) through the volume of interest is simulated. Along its way, the particle may interact with the matter through which it is passing, e.g. through Compton scattering (for photons) or Coulomb scattering (for electrons and protons). For a dose calculation, for each interaction that is simulated, the energy balance is calculated of the energy of the 'incoming' particle(s) minus the energy of the 'outgoing' one(s). To calculate the dose in a particular volume (voxel), the contributions from all interactions taking place inside the volume are added, and divided by the mass in the volume.

For many years it has been realised that full Monte Carlo simulations of the radiotherapy dose delivery process should further improve calculation accuracy. Due to limitations in computing power, however, this was never a realistic option in a clinical setting. Today, available computing power may still not allow for full Monte Carlo simulations in clinical practice.

Given the random nature of Monte Carlo dose calculations, the resulting dose distributions are composed of noisy dose data and associated statistical uncertainty data (noise), which prevents making reliable clinical decisions within short time spans desirable for using or implementing such resulting dose distributions as a setting in a radiation treatment system for the purpose of radiation therapy in a target region or region of interest in, for example, a human or animal body.

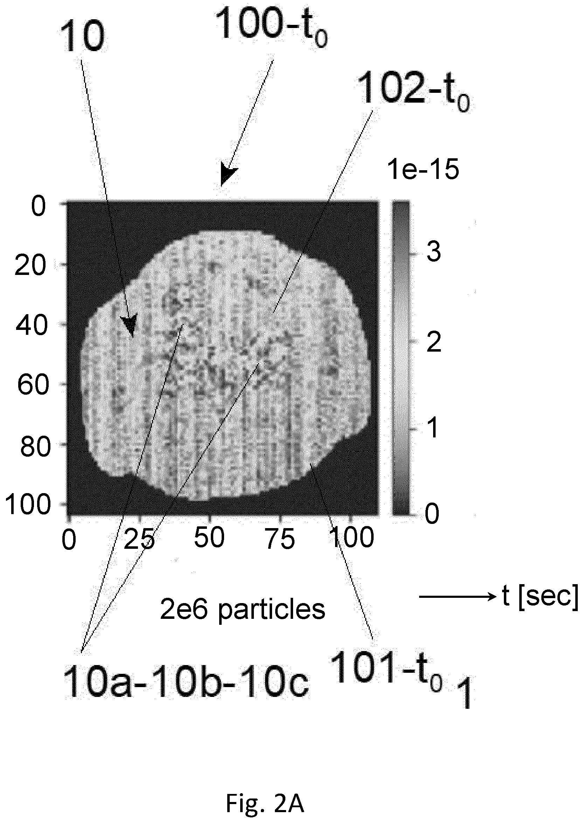

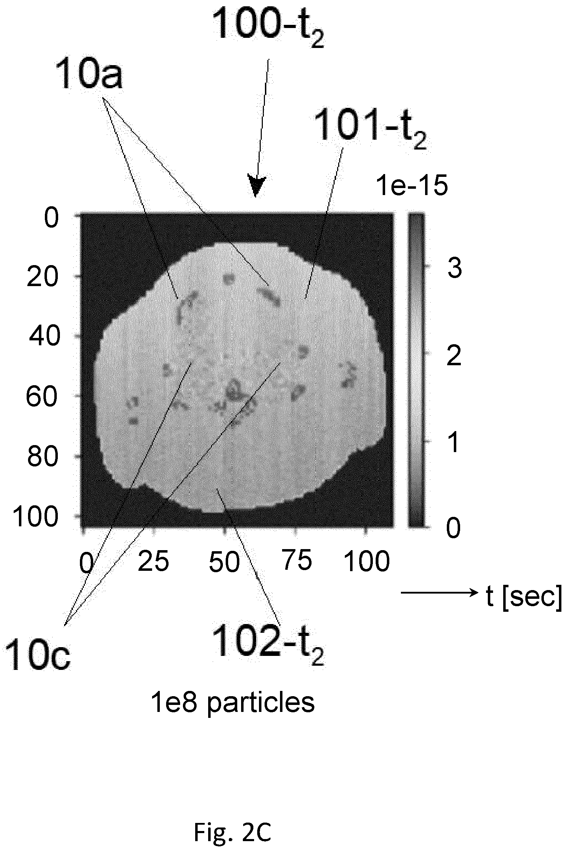

A drawback of present Monte Carlo dose calculations is depicted in Figures 2A-2D, showing four examples of the same radiation dose distribution 100 of a radiation beam passing through a region of interest 10, the radiation dose distributions 100 being calculated at different time moments t₀, t₁, t₂ and t_{INF} over time, and are thus indicated with time-referenced numerals 100-t₀, 100-t₁, 100-t₂ and 100-t_{INF}.

Also in Figures 2A-2D, specific locations of interest 10a-10b-10c are marked within the region of interest 10, in particular the spine 10a, several rib structures 10b and the lungs 10c are visible in the several radiation dose distributions.

Note, that the time references t₀ > t₁ > t₂ > t_{INF}, with 100-t₀ representing the radiation dose distribution being calculated using Monte Carlo simulations after a significant short time period t₀ (approx. after 60 seconds since the start of the Monte Carlo simulation), and with 100-t_{INF} representing the radiation dose distribution being calculated using Monte Carlo simulations after a significant long, comparable with an infinite, calculation time period t_{INF} (approx. 45 minutes).

Due to the statistical nature of Monte Carlo simulations, the radiation dose distribution 100 contains next to noisy dose data 101 also associated uncertainty data 102. In radiation dose distribution 100-t₀ the associated uncertainty data 102-t₀ is dominating and spoiling the noisy dose data 101-t₀. This spoiling of the noisy dose data 101-t₀ is clearly visible in Figure 2A in the form of a scatter super-positioned or overlaying the noisy dose data 101-t₀, in which latter data set the specific locations of interest, such as the spine 10a, several rib structures 10b and the lungs 10c are barely visible. Accordingly, the radiation dose distribution 100-t₀ is clearly unsuitable for making a reliable clinical decision for the purpose of radiation therapy in the actual region of interest 10 of the target object 10, here a rodent.

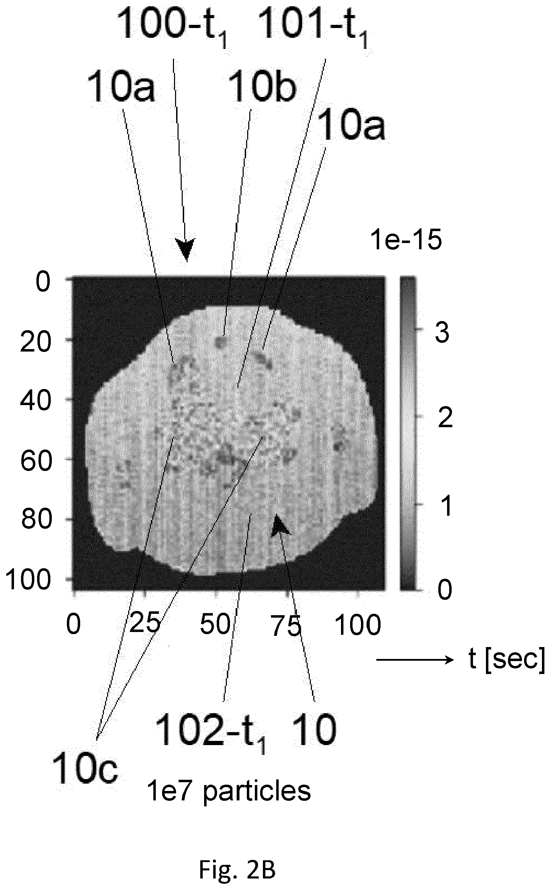

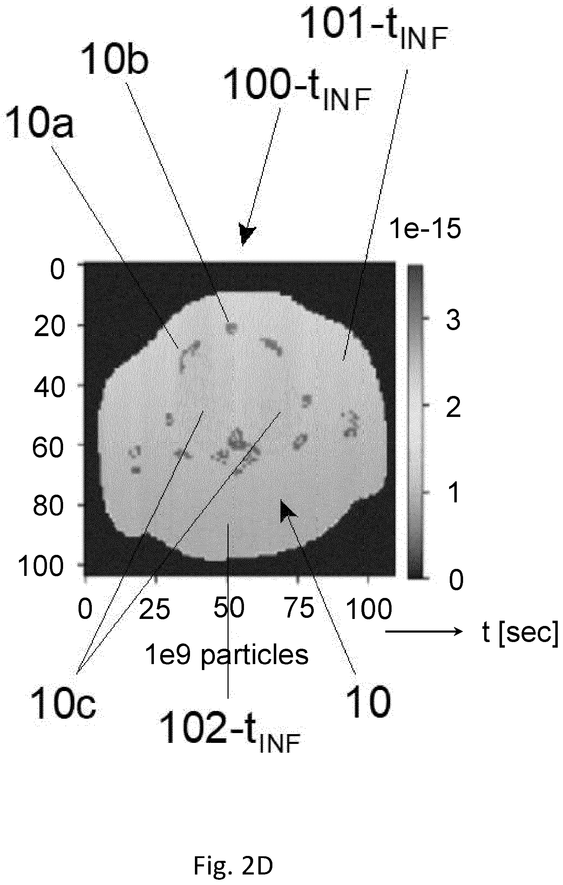

The dominance of the associated uncertainty data 102 will lessen when Monte Carlo simulations are performed over longer periods of time. Figures 2A-2D depict the Monte Carlo simulations of the radiation dose distributions over time t, and when reviewing Figures 2B-2D with Figure 2A, it is clear that in the subsequent radiation dose distributions calculated at t=t₁, t=t₂ and t=t_{INF}, with t₀ > t₁ > t₂ > t_{INF}, the noisy dose data 101-tₓ (with x = 1 or 2 of INF) is more and more dominant and less and less noisy, whereas the associated uncertainty data 102-tₓ (with x = 1 or 2 of INF) becomes more and more less dominant and is nearly absent at t = t_{INF}. In radiation dose distribution 100-t_{INF}, the noisy dose data 101-t_{1NF} is dominant and far less noisy as the associated uncertainty data 102-t_{INF} is nearly absent. In Figure 2D the specific locations of interest, such as the spine 10a, several rib structures 10b and the lungs 10c are clearly visible and accordingly, the radiation dose distribution 100-t_{INF} is far more suitable for making a reliable clinical decision for the purpose of performing radiation therapy.

Accordingly, the radiation dose distribution 100-t_{INF} provides the optimal, most accurate and desirable representation of a radiation dose distribution of a radiation beam passing the region of interest and is thus the most desirable starting point for e.g. radiation therapy treatments.

However, the radiation dose distribution 100-t_{INF} is obtained after a significant long simulation time t_{INF} of more than 30 minutes, which time period can be considered as 'infinite' under medical treatment conditions. Accordingly, such calculation times are unsuitable for real-time treatment practises, and it prevents making reliable clinical decisions within short time spans desirable for using or implementing such resulting dose distributions as a setting in a radiation treatment system for the purpose of radiation therapy in a target region or region of interest in, for example, a human or animal body.

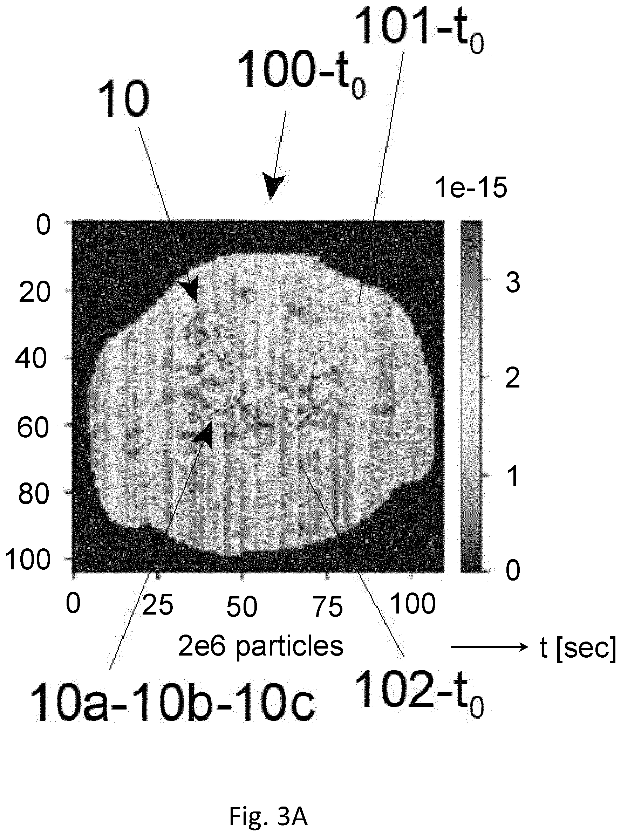

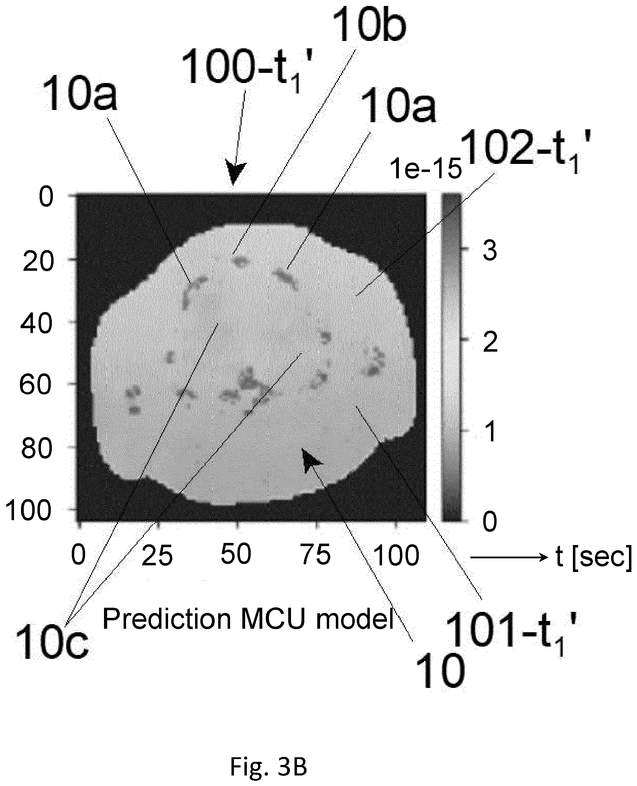

Figures 3A and 3B disclose a radiation dose distribution calculated using Monte Carlo simulations with the use of a computer implemented method for calculating and displaying such a radiation dose distribution according to the invention, and executed by an apparatus according to the invention.

Unlike the radiation dose distribution calculations as depicted in Figures 2A-2D, Figure 3A depicts a radiation dose distribution 100-t₀ being calculated with the computer implemented method according to the invention after a significant short time period t₀ (approx. after no more than 5 seconds since the start of the Monte Carlo simulation using the computer implemented method according to the invention), and 100-t,' represents the radiation dose distribution being calculated using the computer implemented method according to the invention after a somewhat longer calculation time period t₁' (approx. after no more than 15 seconds).

Accordingly, it is noted that the radiation dose distribution 100-t₁ depicted in Figure 2B contains a far more dominant part of the associated uncertainty data 102-t₁, in the form of a scatter super-positioned or overlaying the noisy dose data 101-t₁ whereas the radiation dose distribution 100-t₁' depicted in Figure 3B contains far less, or nearly no associated uncertainty data 102-t₁', and is more or less conformal to the radiation dose distribution 100-t_{INF} of Figure 2D, which radiation dose distribution is obtained after a significant long (nearly infinite) simulation time t_{INF} of more than 30 minutes using Monte Carlo simulations according to the state of the art.

Similarly, in Figure 3B the specific locations of interest, such as the spine 10a, several rib structures 10b and the lungs 10c are far more visible in the radiation dose distribution 100-t₁' (calculated at time t=t₁') compared to the corresponding locations of interest 10a-10b-10c in the radiation dose distribution 100-t₀ of Figure 3A (calculated at time t=t₀). Similarly, the radiation dose distribution 100-t₁' is far more suitable for making a reliable clinical decision for the purpose of performing radiation therapy.

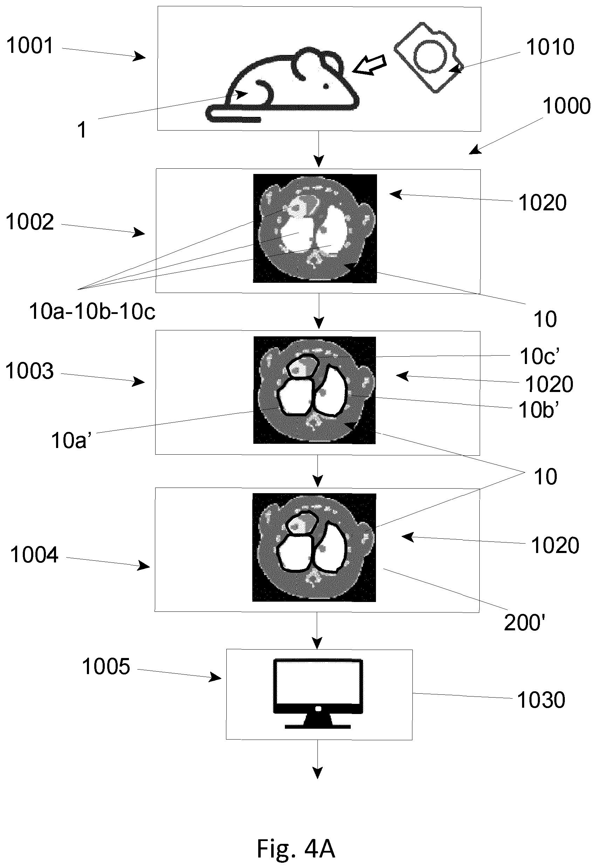

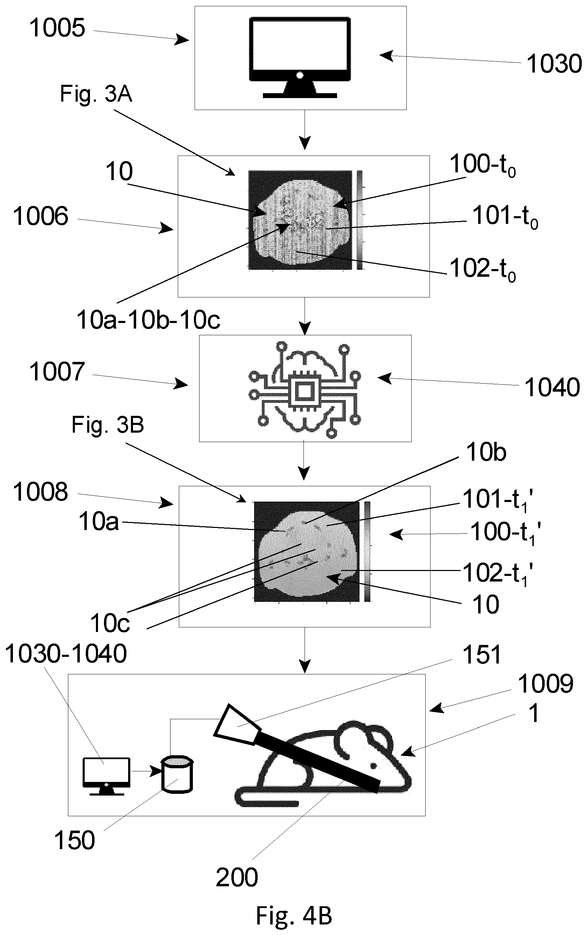

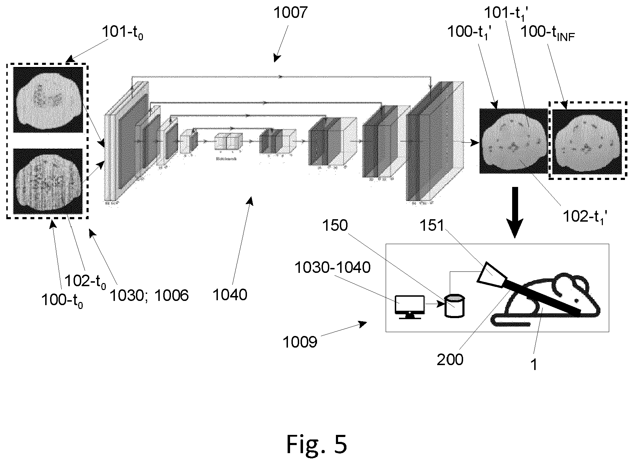

Given the fact that the radiation dose distribution 100-t₁' depicted in Figure 3B contains far less, or nearly no associated uncertainty data 102-t₁' and is being calculated during a significant short time interval using Monte Carlo simulations with the computer implemented method according to the invention, this allows for making reliable clinical decisions within shorter time spans desirable for using or implementing such resulting dose distributions as a setting in a radiation treatment system for the purpose of radiation therapy in a target region or region of interest in, for example, a human or animal body
Such radiation dose distributions 100-t₁' depicted in Figure 3B are being calculated with a computer implemented method according to the invention, which is being executed by an apparatus 1000 according to the invention, an example of both the computer implemented invention and the apparatus 1000 being depicted in Figures 4A and 4B, with a detailed example being depicted in Figure 5.

According to the invention, the computer implemented method calculates and displays, preferable within a computer-generated display of a display device 1020, a radiation dose distribution of a radiation beam 200' using Monte Carlo simulations passing through a target region or region of interest 10 within a target object 1. In this example, the target object 1 is an animal body, e.g. a rodent. It is however noted that both the apparatus 1000 as well as the computer implemented method according to the invention can equally be implemented on a human body or on a phantom body, the latter specifically being used for test purposes.

With the computer implemented method comprising, in a first step 1001 a set of three-dimensional image data is obtained, which set of three-dimensional image data represent the region of interest 10 in the target object 1. The set of three-dimensional image data is also indicated as digital region of interest data representing the simulated region of interest 10 of the target object 1 and can be acquired by means of suitable imaging techniques, such as CT- and/or MRI-scanning systems, indicated with reference numeral 1010.

In particular, it should be noted that in an example such CT- and/or MRI-scanning systems 1010 can be part of the apparatus 1000 according to the invention. However, in another example such CT- and/or MRI-scanning systems 1010 are independent means for acquiring such digital region of interest data representing the simulated region of interest 10 of the target object 1, which digital region of interest data can be stored on suitable storage means, which are part of or can be accessed by the apparatus 1000 according to the invention.

In either example, the simulated region of interest 10 is part of a real target object 1 such as a human or animal body for the purpose of an actual radiotherapy treatment.

In a next step 1002 of the computer implemented method according to the invention, the acquired digital region of interest data is being processed and converted into suitable display signals causing the display device 1020 of the apparatus 1000 of the invention to display the three-dimensional image data set of the region of interest 10 on the display device 1020.

Next, in step 1003, one or more selected locations of interest 10a-10b-10c in the displayed three-dimensional image set are identified, preferably by means of a user selection performed by an user, such as a physician or other medically skilled person operating the apparatus 1000 according to the invention. The identification of the one or more selected locations of interest 10a-10b-10c is preferably based on user interaction between the user and the three-dimensional image data set on the display device 1020 via keyboard or mouse pointer input. The user selection is indicated with reference numerals 10a'-10b'-10c', which identify and characterize the outer contour dimensions of the one or more selected locations of interest 10a-10b-10c (spine, lungs, other organs or bone structures).

In step 1004 of the method according to the invention, the apparatus 1000 is arranged in calculating with the use of radiation dose calculation means 1030 a radiation dose distribution 100 using Monte Carlo simulation. The radiation dose distribution thus calculated belongs to a calculated and simulated radiation beam 200', which passes through the simulated region of interest 10, whilst taking the one or more selected locations of interest 10a-10b-10c (10a'-10b'-10c') into account.

As outlined above, a radiation dose distribution 100 thus calculated is composed of noisy dose data 101 and associated uncertainty data 102.

In next steps 1006 and 1007 of the method according to the invention, see Figure 4B, the radiation dose distribution 100-t₀ thus calculated is being denoised for the associated uncertainty data 102 with the use of one or more trained machine learning algorithms 1040, thereby generating in a final step 1008 a denoised radiation dose distribution 100-t₁'.

By denoising the calculated radiation dose data distribution 100 as obtained using Monte Carlo simulations for the associated uncertainty data set 102 using one or more trained machine learning algorithms 1040, a denoised radiation dose distribution 100-t₁' with barely any associated uncertainty data 102-t₁' can be generated with a significant gain in calculation time, and accordingly the computer implemented method can be used for performing real-time radiation therapy treatments.

Accordingly, the apparatus 1000 comprising the radiation dose calculation means 1030 implementing the one or more trained machine learning algorithms 1040 is further arranged in controlling in a method step 1009 a dose planning software program loaded in a real-time radiation treatment planning software system 150 with a radiation emitter 151, the latter being implemented for generating a radiation beam 200 conformal to the simulated beam 200' as calculated.

By controlling the real-time radiation treatment planning software system 150-151 in accordance with the simulated radiation dose distribution 100-t₁' thus calculated within a short time interval with the method and apparatus according to the invention, radiation therapy of a pre-selected anatomical portion 10 of an animal body 1 (or human body or a phantom body) can be performed in real-time.

For a final check and for allowing the user to make a reliable clinical decision the generated denoised radiation dose data distribution 100-t₁' is being displayed within the three-dimensional image data set of the region of interest 10 on the display device 1020.

The denoised radiation dose distribution 100-t₁' is being denoised for the associated uncertainty data 102 with the use of one or more trained machine learning algorithms 1040. In preferred examples, the one or more machine learning algorithms 1040 are selected from the group exemplified by but not limited to an artificial neural network, a decision tree, a regression model, a k-nearest neighbour model, a partial least squares model, a support vector machine, or an ensemble of the models that are integrated to define an algorithm.

Preferably, the one or more machine learning algorithms 1040 is a computer-implemented artificial neural network. An example of an apparatus implementing a computer-implemented artificial neural network 1040 is depicted in Figure 5.

For a proper calculation of a denoised radiation dose distribution 100-t₁' suitable for implementation in real-time radiation therapy treatments on a target body 1, the computer-implemented artificial neural network 1040 needs to be trained in advance in order to denoise a radiation dose distribution 100-t₀ for its associated uncertainty data 102-t₀.

Accordingly, for training the computer-implemented artificial neural network 1040, the computer implemented method further implementing three training steps.

A first training step comprises the inputting, to the computer-implemented artificial neural network 1040, of several sets of training data. It is desirable to train the artificial neural network 1040 with multiple sets of training data. Each set of training data being inputted, at least comprises training region of interest data 10_{TR}, training noisy dose data 101_{TR}, training associated uncertainty data 102_{TR} and training denoised dose data 100_{TR}-t_{INF}. With the training denoised dose data 100_{TR}-t_{INF} is meant a dose data distribution 100_{TR}-t_{INF} calculated using Monte Carlo simulation after a significant long calculation time period t_{INF} (approx. 45 minutes , considered infinitive under medical treatment conditions), in which the associated uncertainty data 102 is nearly absent, see Figure 2D.

The above four subsets of the set of training data characterize at least one training radiation dose distribution 100_{TR}-t₀ of a radiation beam 200_{TR} passing through the training region of interest 10_{T} and one or more selected training locations of interest 10a_{TR}-10b_{TR}-10c_{TR} in the training region of interest 10_{TR}. The result of this step is the trained artificial neural network which can be used for subsequent denoising of a subsequent radiation dose data distribution 100-t₀ calculated using Monte Carlo simulations according to the invention.

To optimize the trained artificial neural network, in a next training step, so-called sets of test data are applied to the computer-implemented artificial neural network 1040. Similarly, each set of test data comprise subsets comprising test region of interest data 10_{TEST}, test noisy dose data 101_{TEST} and test associated uncertainty data 102_{TEST} and these sets characterize a test radiation dose distribution 100_{TEST}-t₀ of a radiation beam 200_{TEST} passing through a test region of interest 10_{TEST} and one or more selected test locations of interest 10a_{TEST}-10b_{TEST}-10c_{TEST} in the test region of interest 10_{TEST}.

It should be noted that the digital region of interest data 10 (belonging to either training data set TR and test data test TEST) represent a stored 3D image data simulation of the region of interest 10_{TR}-10_{TEST} of the target object 1_{TR}-1_{TEST}. The digital region of interest data 10_{TR} and 10_{TEST} include parameters or data relating to the outer contours and the structure (density, several materials and tissues, etc.) of the target body 1_{TR}-1_{TEST}, the exact locations of certain locations of interest 10a-10b-10c within the region of interest 10_{TR}-10_{TEST}, etc.. The locations of interest can encompass internal organs, such as lungs, heart, intestines, urinary bladder, reproductive organs, etc. as well as the bone structure. Another, often important location of interest within the region of interest can be tumour tissue, in particular its location and its size inside the target body.

These region of interest data 10_{TR}-10_{TEST} together with specific parameters related to the target body 1_{TR}-1_{TEST}, such as the gender, age and dimensions of the patient in the event the target body pertains to a human or animal body, can at a later stage used for a proper and reliable calculation of an actual denoised dose data 100-t₁' for use by a dose planning software program loaded in the radiation treatment planning software system 150-151.

Preferably, the artificial neural network is trained on the basis of machine learning techniques, in particular deep learning, for example by back propagation. The inputted training data TR used can be classified in three types of data sets, that is noisy dose data set 101, and the associated statistical uncertainty data (noise) set 102, both data sets obtained using Monte Carlo simulations over a limited or short calculation time period t₀, as well as an associated denoised dose data set 100-t₁'.

In an example, the computer-implemented artificial neural network 1040 is a deep learning neural network comprising 2D, and/or 3D convolutional layers, and/or recurrent layers as depicted in Figure 5.

According to the disclosure and as depicted in Figures 4A-4B and 5, the apparatus for calculating and displaying a radiation dose distribution 100-t₁' of a radiation beam 200 using Monte Carlo simulations passing through a region of interest 10 within a target object 1 comprises image data processing means 1020 for obtaining a set of image data representing the region of interest 10, selecting means for selecting one or more selected locations of interest 10a-10b-10c/10a'-10b'-10c' within the three-dimensional image set 10, radiation dose calculation means 1030 for calculating a radiation dose data distribution 100t₀ using Monte Carlo simulation of a simulated radiation beam 200' passing through the region of interest 10, wherein the calculated radiation dose data distribution 100-t₀ being composed of noisy dose data 101 and associated uncertainty data 102 and wherein the radiation dose calculation means 1030 are further configured in denoising the radiation dose data distribution 100-t₀ for the associated uncertainty data 102 using one or more trained machine learning algorithms 1040, thereby generating a denoised radiation dose distribution 100-t₁'.

Accordingly, as with the computer implemented method according to the disclosure, as the apparatus 1000 is capable in denoising the calculated radiation dose data distribution 100-t₀ as obtained using Monte Carlo simulations for the associated uncertainty data set 102 using one or more trained machine learning algorithms 1040, a denoised radiation dose distribution 100-t₁' can be generated with a significant gain in calculation time, and accordingly the computer implemented method can be used for performing real-time radiation therapy treatments.

Herewith, expensive and specialized radiation therapy treatment equipment requiring intensive computing power is avoided. The apparatus according to an example of the disclosure can be simplified as it requires less complex equipment, and it allows for a significant speed up of the dose distribution calculations, whilst maintaining dose distribution accuracy and thus still making reliable clinical decisions.

In an further example, the apparatus also comprises a display device 1020 for displaying the three-dimensional image data set of the region of interest 10 and the denoised radiation dose data distribution 100-t₁' in the three-dimensional image data set.

It should be noted that the apparatus implementing the one or more machine learning algorithms as a computer-implemented artificial neural network can be trained as outlined above. In particular the radiation dose calculation means 1030 comprises a training unit to train the computer-implemented artificial neural network 1030, the training unit being configured to input, to the computer-implemented artificial neural network 1040, several sets of training data. Each set of training data being inputted, at least comprises training region of interest data 10_{TR}, training noisy dose data 101_{TR}, training associated uncertainty data 102_{TR} and training denoised dose data 100_{TR}-t_{INF}, the latter calculated using Monte Carlo simulation after a significant long calculation time period t_{INF} (approx. 45 minutes), in which the associated uncertainty data 102 is nearly absent, see Figure 2D. The above four sets of training data characterize at least one training radiation dose distribution 100_{TR}-t₀ of a radiation beam 200_{TR} passing through the training region of interest 10_{T} and one or more selected training locations of interest 10a_{TR}-10b_{TR}-10c_{TR} in the training region of interest 10_{TR}.

Additionally, so-called sets of test data are applied by the training unit to the computer-implemented artificial neural network 1040. The sets of test data comprise test region of interest data 10_{TEST}, test noisy dose data 101_{TEST} and test associated uncertainty data 102_{TEST} and these sets characterize a test radiation dose distribution 100_{TEST}-t₀ of a radiation beam 200_{TEST} passing through a test region of interest 10_{TEST} and one or more selected test locations of interest 10a_{TEST}-10b_{TEST}-10c_{TEST} in the test region of interest 10_{TEST}.

Furthermore, the training unit is capable of analyzing each applied test radiation dose distribution 100_{TEST}-t₀ using the at least one training radiation dose distribution 100_{TR}-t₀ in order to generate the corresponding denoised radiation dose distribution 100_{TEST}-t₁' for each test noisy dose data 101_{TEST} and test associated uncertainty data 102_{TEST}.

This generated denoised radiation dose distribution 100_{TEST}-t₁', conforms in a most optimal way to a corresponding training denoised dose data set 100_{TR}-t_{INF} already present in the trained artificial neural network and having nearly absent associated uncertainty data 102, and the result of these optimizing steps is the fine-tuned artificial neural network.

Next, for calculating a denoised radiation dose distribution 100-t₁' of a radiation beam 200 using the computer implemented method and apparatus according to the invention, the following data set is to be inputted:
- region of interest data 10 including parameters or data relating to the outer contours and the structure (density, different materials and tissues, etc.) of the target body 1, the exact locations of certain locations of interest 10a-10b-10c within the region of interest 10, optionally the location and size of any tumor tissue inside the target body (in the event of a human or animal body;
- a calculated radiation dose data distribution 100-t₀ at time interval t = t₀ of a radiation beam passing through the region of interest taking the region of interest data 10 into account, calculated radiation dose data distribution 100-t₀ being composed of noisy dose data 101-t₀ and associated uncertainty data 102-t₀.

The apparatus implementing the computer implemented method according to the invention that is implementing a completely trained neural network as described above, will calculate (denoise) the radiation dose data distribution 100-t₀ for its associated uncertainty data 102-t₀ using the trained computer-implemented artificial neural network 1040, which is trained with multiple data sets of training dose data distributions 100_{TR}. The calculated radiation dose distribution 100-t₀ is analysed or compared with the database of these multiple data sets of training dose data distributions 100_{TR}. Based on that analysis or comparison the computer-implemented artificial neural network 1040 will output a denoised radiation dose distribution 100-t₁' obtained after a relatively short calculation time t₁' and having nearly absent associated uncertainty data 102. The denoised radiation dose distribution 100-t₁' conforms in a most optimal way to a corresponding dose data distribution 100-t_{INF} (being depicted next to the denoised radiation dose distribution 100-t₁' as the so-called 'golden standard'), however the latter could only be calculated after a significant longer calculation time t_{INF} using state of art Monte Carlo simulations.

In a further example, the computer implemented method of the present disclosure can be embodied in a computer program or product, which computer program or product comprises computer-coded instructions which, when the computer program or product program is executed by a computer, such as a laptop or a computer, cause the computer to carry out steps of the computer implemented method disclosed herein.

In a particular embodiment, a computer-readable storage medium is proposed comprising computer-coded instructions stored therein, which computer-coded instructions, when executed by a computer, causes the computer to carry out steps of the computer implemented method disclosed in this application. Such computer-readable storage medium can be a (solid-state) hard drive, a USB drive, or a (digital) optical disc.

## Claims

1. A computer implemented method for calculating and displaying, within a computer-generated display of a display device (1020), a radiation dose distribution of a radiation beam (200') using Monte Carlo simulations passing through a region of interest (10) within a target object (1), the computer implemented method comprising at least the steps of:
i) obtaining three-dimensional image data set representing the region of interest (10) in the target object (1);
ii) generating a display signal causing the display device (1020) to display the three-dimensional image data set of the region of interest (10) on the display device (1020),
iii) receiving a user selection, based on user interaction with the three-dimensional image data set on the display device (1020), the user selection identifying one or more selected locations of interest (10a, 10b, 10c) within the region of interest (10) in the displayed three-dimensional image data set;
iv) calculating, using radiation dose calculation means (1030), a radiation dose distribution using Monte Carlo simulations of a radiation beam (200') passing through the region of interest (10) taking the one or more selected locations of interest (10a, 10b, 10c) into account, the radiation dose distribution being composed of noisy dose data (101) and associated uncertainty data (102);
v) denoising the radiation dose data distribution for the associated uncertainty data (102) with a computer-implemented artificial neural network (1040), thereby generating a denoised radiation dose distribution (100-t1'),
wherein, for training the computer-implemented artificial neural network (1040), the computer implemented method further comprises the steps of:
A) inputting, to the computer-implemented artificial neural network (1040), training region of interest data (10TR), training noisy dose data (101TR), training associated uncertainty data (102TR) and training denoised dose data (100TR-tINF) characterizing at least one training radiation dose distribution of a radiation beam (200TR) passing through the training region of interest (10TR) as well as one or more known selected locations of interest in the training region of interest;
B) applying, to the computer-implemented artificial neural network (1040), test region of interest data (10TEST), test noisy dose data (101TEST) and test associated uncertainty data (102TEST) characterizing a test radiation dose distribution of a radiation beam (200TEST) passing through a test region of interest (10TEST) and one or more selected test locations of interest in the test region of interest; and
C) analyzing each applied test radiation dose distribution using the at least one training radiation dose distribution to generate a denoised radiation dose distribution for each test noisy dose data and test associated uncertainty data.

2. The computer-implemented method according to claim 1, wherein the computer implemented method further comprises the step of:
vi) displaying, within the three-dimensional image data set of the region of interest (10) displayed in the display device (1020), the generated denoised radiation dose data distribution (100-t1').

3. The computer-implemented method according to any one or more of the claims 1 to 2, wherein the computer-implemented artificial neural network (1040) is a deep learning neural network comprising 2D and/or 3D convolutional layers, and/or recurrent layers.

4. An apparatus (1000) for calculating and displaying a radiation dose distribution of a radiation beam (200') using Monte Carlo simulations passing through a region of interest (10) within a target object (1), the apparatus comprises:
- image data processing means for obtaining a three-dimensional image data set representing the region of interest (10);
- selecting means for selecting one or more selected locations of interest (10a, 10b, 10c) within the three-dimensional image data set;
- radiation dose calculation means (1030) for calculating a radiation dose data distribution using Monte Carlo simulations of a simulated radiation beam (200') passing through the region of interest (10), wherein the calculated radiation dose data distribution is composed of noisy dose data (101) and associated uncertainty data (102), and
wherein
the radiation dose calculation means (1030) are further configured in denoising the radiation dose data distribution for the associated uncertainty data (102) with a computer-implemented artificial neural network (1040), thereby generating a denoised radiation dose distribution (100-t1'), wherein the radiation dose calculation means (1030) comprises a training unit configured to train the computer-implemented artificial neural network (1040), the training unit being configured to:
A) input, to the computer-implemented artificial neural network (1040), training region of interest data (10TR), training noisy dose data (101TR), training associated uncertainty data (102TR) and training denoised dose data (100TR-tINF) characterizing at least one training radiation dose distribution of a radiation beam (200TR) passing through the training region of interest (10TR) and one or more known selected training locations of interest in the training region of interest;
B) apply, to the computer-implemented artificial neural network (1040), test region of interest data (10TEST), test noisy dose data (101TEST) and test associated uncertainty data (102TEST) characterizing a test radiation dose distribution of a radiation beam (200TEST) passing through a test region of interest (10TEST) and one or more selected test target locations in the test region of interest; and
C) analyze each applied test radiation dose distribution using the at least one training radiation dose distribution to generate a denoised radiation dose distribution for each test noisy dose data and test associated uncertainty data.

5. The apparatus (1000) according to claim 4, further comprising a display device (1020) for displaying the three-dimensional image data set of the region of interest (10) and the denoised radiation dose data distribution (100-t1') in the three-dimensional image data set.

6. The apparatus (1000) according to any one or more of the claims 4 to 5, wherein the computer-implemented artificial neural network (1040) is a deep neural network comprising 2D, and/or 3D convolutional layers, and/or recurrent layers.

7. A computer program or product comprising instructions which, when the program is executed by a computer, cause the computer to carry out steps of the computer implemented method according to any one or more of the claims 1-3.

8. A computer-readable storage medium comprising instructions which, when executed by a computer, cause the computer to carry out steps of the computer implemented method according to any one or more of the claims 1-3.

## Patentansprüche

1. Computerimplementiertes Verfahren zum Berechnen und Anzeigen, innerhalb einer computergenerierten Anzeige eines Anzeigegeräts (1020), einer Strahlungsdosisverteilung eines Strahlenbündels (200^{'}) unter Verwendung von Monte-Carlo-Simulationen, das durch eine Region von Interesse (10) innerhalb eines Zielobjekts (1) verläuft, wobei das computerimplementierte Verfahren mindestens die folgenden Schritte umfasst:
i) Erhalten eines dreidimensionalen Bilddatensatzes, der die Region von Interesse (10) in dem Zielobjekt (1) darstellt;
ii) Erzeugen eines Anzeigesignals, das das Anzeigegerät (1020) veranlasst, den dreidimensionalen Bilddatensatz der Region von Interesse (10) auf dem Anzeigegerät (1020) anzuzeigen,
iii) Empfangen einer Benutzerauswahl basierend auf einer Benutzerinteraktion mit dem dreidimensionalen Bilddatensatz auf dem Anzeigegerät (1020), wobei die Benutzerauswahl einen oder mehrere ausgewählte Orte von Interesse (10a, 10b, 10c) innerhalb der Region von Interesse (10) in dem angezeigten dreidimensionalen Bilddatensatz identifiziert;
iv) Berechnen, unter Verwendung von Strahlungsdosisberechnungsmitteln (1030), einer Strahlungsdosisverteilung unter Verwendung von Monte-Carlo-Simulationen eines Strahlenbündels (200^{'}), das durch die Region von Interesse (10) verläuft, unter Berücksichtigung des einen oder der mehreren ausgewählten Orte von Interesse (10a, 10b, 10c), wobei die Strahlungsdosisverteilung aus verrauschten Dosisdaten (101) und zugehörigen Unsicherheitsdaten (102) besteht;
v) Entrauschen der Strahlungsdosisdatenverteilung für die zugehörigen Unsicherheitsdaten (102) mit einem computerimplementierten künstlichen neuronalen Netzwerk (1040), wodurch eine entrauschte Strahlungsdosisverteilung (100-t1^{'}) erzeugt wird, wobei
zum Trainieren des computerimplementierten künstlichen neuronalen Netzwerks (1040) das computerimplementierte Verfahren ferner die folgenden Schritte umfasst:
A) Eingeben, in das computerimplementierte künstliche neuronale Netzwerk (1040), von Daten der Trainingsregion von Interesse (10TR), verrauschten Trainingsdosisdaten (101TR), zugehörigen Trainingsunsicherheitsdaten (102TR) und entrauschten Trainingsdosisdaten (100TR-tlNF), die mindestens eine Trainingsstrahlungsdosisverteilung eines Strahlenbündels (200TR) charakterisieren, das durch die Trainingsregion von Interesse (10TR) sowie durch einen oder mehrere bekannte ausgewählte Orte von Interesse in der Trainingsregion von Interesse verläuft;
B) Anwenden, auf das computerimplementierte künstliche neuronale Netzwerk (1040), von Daten einer Testregion von Interesse (10TEST), verrauschten Testdosisdaten (101TEST) und zugehörigen Testunsicherheitsdaten (102TEST), die eine Teststrahlungsdosisverteilung eines Strahlenbündels (200TEST) charakterisieren, das durch eine Testregion von Interesse (10TEST) und einen oder mehrere ausgewählte Testorte von Interesse in der Testregion von Interesse verläuft; und
C) Analysieren jeder angewendeten Teststrahlungsdosisverteilung unter Verwendung der mindestens einen Trainingsstrahlungsdosisverteilung, um eine entrauschte Strahlungsdosisverteilung für jede verrauschten Testdosisdaten und zugehörigen Testunsicherheitsdaten zu erzeugen.

2. Computerimplementiertes Verfahren nach Anspruch 1, wobei das computerimplementierte Verfahren ferner den folgenden Schritt umfasst:
vi) Anzeigen, innerhalb des dreidimensionalen Bilddatensatzes der Region von Interesse (10), die in dem Anzeigegerät (1020) angezeigt wird, der erzeugten entrauschten Strahlungsdosisdatenverteilung (100-t1^{'}).

3. Computerimplementiertes Verfahren nach einem oder mehreren der Ansprüche 1 bis 2, wobei das computerimplementierte künstliche neuronale Netzwerk (1040) ein neuronales Deep-Learning-Netzwerk ist, das 2D- und/oder 3D-Faltungsschichten und/oder rekurrente Schichten umfasst.

4. Vorrichtung (1000) zum Berechnen und Anzeigen einer Strahlungsdosisverteilung eines Strahlenbündels (200^{'}) unter Verwendung von Monte-Carlo-Simulationen, das durch eine Region von Interesse (10) innerhalb eines Zielobjekts (1) verläuft, wobei die Vorrichtung Folgendes umfasst:
- Bilddatenverarbeitungsmittel zum Erhalten eines dreidimensionalen Bilddatensatzes, der die Region von Interesse (10) darstellt;
- Auswählmittel zum Auswählen eines oder mehrerer ausgewählter Orte von Interesse (10a, 10b, 10c) innerhalb des dreidimensionalen Bilddatensatzes:
- Strahlungsdosisberechnungsmittel (1030) zum Berechnen einer Strahlungsdosisdatenverteilung unter Verwendung von Monte-Carlo-Simulationen eines simulierten Strahlenbündels (200^{'}), das durch die Region von Interesse (10) verläuft, wobei die berechnete Strahlungsdosisdatenverteilung aus verrauschten Dosisdaten (101) und zugehörigen Unsicherheitsdaten (102) besteht und wobei
die Strahlungsdosisberechnungsmittel (1030) ferner dazu konfiguriert sind, die Strahlungsdosisdatenverteilung für die zugehörigen Unsicherheitsdaten (102) mit einem computerimplementierten künstlichen neuronalen Netzwerk (1040) zu entrauschen, wodurch eine entrauschte Strahlungsdosisverteilung (100-t1^{'}) erzeugt wird, wobei
die Strahlungsdosisberechnungsmittel (1030) eine Trainingseinheit umfassen, die dazu konfiguriert ist, das computerimplementierte künstliche neuronale Netzwerk (1040) zu trainieren, wobei die Trainingseinheit zu Folgendem konfiguriert ist:
A) Eingeben, in das computerimplementierte künstliche neuronale Netzwerk (1040), von Daten der Trainingsregion von Interesse (10TR), verrauschten Trainingsdosisdaten (101TR), zugehörigen Trainingsunsicherheitsdaten (102TR) und entrauschten Trainingsdosisdaten (100TR-tlNF), die mindestens eine Trainingsstrahlungsdosisverteilung eines Strahlenbündels (200TR) charakterisieren, das durch die Trainingsregion von Interesse (10TR) und durch einen oder mehrere bekannte ausgewählte Trainingsorte von Interesse in der Trainingsregion von Interesse verläuft;
B) Anwenden, auf das computerimplementierte künstliche neuronale Netzwerk (1040), von Daten einer Testregion von Interesse (10TEST), verrauschten Testdosisdaten (101TEST) und zugehörigen Testunsicherheitsdaten (102TEST), die eine Teststrahlungsdosisverteilung eines Strahlenbündels (200TEST) charakterisieren, das durch eine Testregion von Interesse (10TEST) und einen oder mehrere ausgewählte Testzielorte in der Testregion von Interesse verläuft; und
C) Analysieren jeder angewendeten Teststrahlungsdosisverteilung unter Verwendung der mindestens einen Trainingsstrahlungsdosisverteilung, um eine entrauschte Strahlungsdosisverteilung für jede verrauschten Testdosisdaten und zugehörigen Testunsicherheitsdaten zu erzeugen.

5. Vorrichtung (1000) nach Anspruch 4, ferner umfassend ein Anzeigevgerät (1020) zum Anzeigen des dreidimensionalen Bilddatensatzes der Region von Interesse (10) und der entrauschten Strahlungsdosisdatenverteilung (100-t1^{'}) in dem dreidimensionalen Bilddatensatz.

6. Vorrichtung (1000) nach einem oder mehreren der Ansprüche 4 bis 5, wobei das computerimplementierte künstliche neuronale Netzwerk (1040) ein tiefes neuronales Netzwerk ist, das 2D- und/oder 3D-Faltungsschichten und/oder rekurrente Schichten umfasst.

7. Computerprogramm oder -produkt, umfassend Anweisungen, die, wenn das Programm durch einen Computer ausgeführt wird, den Computer veranlassen, Schritte des computerimplementierten Verfahrens nach einem oder mehreren der Ansprüche 1 bis 3 durchzuführen.

8. Computerlesbares Speichermedium, umfassend Anweisungen, die, wenn sie durch einen Computer ausgeführt werden, den Computer veranlassen Schritte des computerimplementierten Verfahrens nach einem oder mehreren der Ansprüche 1 bis 3 durchzuführen.

## Revendications

1. Procédé mis en œuvre par ordinateur pour calculer et afficher, dans un affichage généré par ordinateur d'un dispositif d'affichage (1020), une distribution de dose de rayonnement d'un faisceau de rayonnement (200') à l'aide de simulations de Monte Carlo traversant une zone d'intérêt (10) dans un objet cible (1), le procédé mis en œuvre par ordinateur comprenant au moins les étapes de :
i) obtention d'un ensemble de données d'image tridimensionnelle représentant la zone d'intérêt (10) dans l'objet cible (1) ;
ii) génération d'un signal d'affichage amenant le dispositif d'affichage (1020) à afficher l'ensemble de données d'image tridimensionnelle de la zone d'intérêt (10) sur le dispositif d'affichage (1020),
iii) réception d'une sélection d'utilisateur, sur la base de l'interaction de l'utilisateur avec l'ensemble de données d'image tridimensionnelle sur le dispositif d'affichage (1020), la sélection d'utilisateur identifiant un ou plusieurs emplacements d'intérêt sélectionnés (10a, 10b, 10c) dans la zone d'intérêt (10) dans l'ensemble de données d'image tridimensionnelle affiché ;
iv) calcul, à l'aide d'un moyen de calcul de dose de rayonnement (1030), d'une distribution de dose de rayonnement à l'aide de simulations de Monte Carlo d'un faisceau de rayonnement (200') traversant la zone d'intérêt (10) en tenant compte desdits un ou plusieurs emplacements d'intérêt sélectionnés (10a, 10b, 10c), la distribution de dose de rayonnement étant composée de données de dose bruitées (101) et de données d'incertitude associées (102) ;
v) débruitage de la distribution de données de dose de rayonnement pour les données d'incertitude associées (102) avec un réseau neuronal artificiel mis en œuvre par ordinateur (1040), ce qui permet de générer une distribution de dose de rayonnement débruitée (100-t1'),
pour entraîner le réseau neuronal artificiel mis en œuvre par ordinateur (1040), ledit procédé mis en œuvre par ordinateur comprenant en outre les étapes de :
A) entrée, dans le réseau neuronal artificiel mis en œuvre par ordinateur (1040), de données de zone d'intérêt d'entraînement (10TR), de données de dose bruitées d'entraînement (101TR), de données d'incertitude associées à l'entraînement (102TR) et de données de dose débruitées d'entraînement (100TR-tlNF) caractérisant au moins une distribution de dose de rayonnement d'entraînement d'un faisceau de rayonnement (200TR) traversant la zone d'intérêt d'entraînement (10TR) ainsi qu'un ou plusieurs emplacements d'intérêt sélectionnés connus dans la zone d'intérêt d'entraînement ;
B) application, au réseau neuronal artificiel mis en œuvre par ordinateur (1040), de données de zone d'intérêt de test (10TEST), de données de dose bruitées de test (101TEST) et de données d'incertitude associées de test (102TEST) caractérisant une distribution de dose de rayonnement de test d'un faisceau de rayonnement (200TEST) traversant une zone d'intérêt de test (10TEST) et un ou plusieurs emplacements d'intérêt de test sélectionnés dans la zone d'intérêt de test ; et
C) analyse de chaque distribution de dose de rayonnement de test appliquée à l'aide de ladite au moins une distribution de dose de rayonnement d'entraînement pour générer une distribution de dose de rayonnement débruitée pour chaque donnée de dose bruitée de test et des données d'incertitude associées à un test.

2. Procédé mis en œuvre par ordinateur selon la revendication 1, ledit procédé mis en œuvre par ordinateur comprenant en outre l'étape de :
vi) affichage, dans l'ensemble de données d'image tridimensionnelle de la zone d'intérêt (10) affiché dans le dispositif d'affichage (1020), de la distribution de données de dose de rayonnement débruitée générée (100-t1').

3. Procédé mis en œuvre par ordinateur selon l'une quelconque ou plusieurs des revendications 1 à 2, ledit réseau neuronal artificiel mis en œuvre par ordinateur (1040) étant un réseau neuronal d'apprentissage profond comprenant des couches convolutives 2D et/ou 3D, et/ou des couches récurrentes.

4. Appareil (1000) permettant de calculer et d'afficher une distribution de dose de rayonnement d'un faisceau de rayonnement (200') à l'aide de simulations de Monte Carlo traversant une zone d'intérêt (10) dans un objet cible (1), l'appareil comprenant :
- un moyen de traitement de données d'image pour obtenir un ensemble de données d'image tridimensionnelle représentant la zone d'intérêt (10) ;
- un moyen de sélection pour sélectionner un ou plusieurs emplacements d'intérêt sélectionnés (10a, 10b, 10c) dans l'ensemble de données d'image tridimensionnelle :
- un moyen de calcul de dose de rayonnement (1030) pour calculer une distribution de données de dose de rayonnement à l'aide de simulations de Monte Carlo d'un faisceau de rayonnement simulé (200') traversant la zone d'intérêt (10), ladite distribution de données de dose de rayonnement calculée étant composée de données de dose bruitées (101) et de données d'incertitude associées (102) et
ledit moyen de calcul de dose de rayonnement (1030) étant en outre conçus pour débruiter la distribution de données de dose de rayonnement pour les données d'incertitude associées (102) avec un réseau neuronal artificiel mis en œuvre par ordinateur (1040), ce qui permet de générer une distribution de dose de rayonnement débruitée (100-t1'), ledit moyen de calcul de dose de rayonnement (1030) comprenant une unité d'entraînement configurée pour entraîner le réseau neuronal artificiel mis en œuvre par ordinateur (1040), ladite unité d'entraînement étant configurée pour :
A) entrer, dans le réseau neuronal artificiel mis en œuvre par ordinateur (1040), des données de zone d'intérêt d'entraînement (10TR), des données de dose bruitées d'entraînement (101TR), des données d'incertitude associées à l'entraînement (102TR) et des données de dose débruitées d'entraînement (100TR-tlNF) caractérisant au moins une distribution de dose de rayonnement d'entraînement d'un faisceau de rayonnement (200TR) traversant la zone d'intérêt d'entraînement (10TR) et un ou plusieurs emplacements d'intérêt d'entraînement sélectionnés connus dans la zone d'intérêt d'entraînement ;
B) appliquer, au réseau neuronal artificiel mis en œuvre par ordinateur (1040), des données de zone d'intérêt de test (10TEST), des données de dose bruitées de test (101TEST) et des données d'incertitude associées de test (102TEST) caractérisant une distribution de dose de rayonnement de test d'un faisceau de rayonnement (200TEST) traversant une zone d'intérêt de test (10TEST) et un ou plusieurs emplacements cibles de test sélectionnés dans la zone d'intérêt de test ; et
C) analyser chaque distribution de dose de rayonnement de test appliquée à l'aide de ladite au moins une distribution de dose de rayonnement d'entraînement pour générer une distribution de dose de rayonnement débruitée pour chaque donnée de dose bruitée de test et des données d'incertitude associées à un test.

5. Appareil (1000) selon la revendication 4, comprenant en outre un dispositif d'affichage (1020) destiné à afficher l'ensemble de données d'image tridimensionnelle de la zone d'intérêt (10) et la distribution de données de dose de rayonnement débruitées (100-t1') dans l'ensemble de données d'image tridimensionnelle.

6. Appareil (1000) selon l'une quelconque ou plusieurs des revendications 4 à 5, ledit réseau neuronal artificiel mis en œuvre par ordinateur (1040) étant un réseau neuronal profond comprenant des couches convolutives 2D, et/ou 3D, et/ou des couches récurrentes.

7. Programme ou produit informatique comprenant des instructions qui, lorsque le programme est exécuté par un ordinateur, amènent l'ordinateur à effectuer les étapes du procédé mis en œuvre par ordinateur selon l'une quelconque ou plusieurs des revendications 1 à 3.

8. Support de stockage lisible par ordinateur comprenant des instructions qui, lorsqu'elles sont exécutées par un ordinateur, amènent l'ordinateur à effectuer les étapes du procédé mis en œuvre par ordinateur selon l'une quelconque ou plusieurs des revendications 1 à 3.
